# EUROPEAN PATENT APPLICATION

(11) **EP 4 123 070 A1**
(43) Date of publication of application: **25.01.2023**
(21) Application number: 22183970.7
(22) Date of filing: 08.07.2022
(51) Int. Cl.: D02G 3/02, A61P 17/18, A61P 31/02, A61Q 19/00, A61Q 19/08, A61F 13/00

(54) **HEMP FIBRE, YARN, HEMP FLAT TEXTILE PRODUCT WITH SKIN CONDITIONING PROPERTIES, CLOTHING AND METHOD OF MANUFACTURING THE FLAT TEXTILE PRODUCT**

(30) Priority: 08.07.2021 PL 43838821
(71) Applicant: Instytut Wlokien Naturalnych i Roslin Zielarskich, 60-630 Poznan (PL)
(72) Inventor: ZIMNIEWSKA, Malgorzata, 62-081 Przezmierowo (PL); ROMANOWSKA, Barbara, 92-305 Lodz (PL); GRYSZCZYNSKA, Agnieszka, 64-400 Miedzychod (PL); PAWLACZYK, Iwona, 60-461 Poznan (PL)
(74) Representative: Twardowska-Czerwinska, Aleksandra

(57) **Abstract**

The subject of the invention is hemp fiber, yarn, hemp flat textile product with properties improving the skin condition, clothing and a method for producing a flat textile product. More specifically, the solution concerns bioactive hemp woven fabrics, knitted fabrics and nonwovens, which improve the skin condition and slow down the visible signs of skin aging when used in clothing. Clothing is made of bioactive materials derived from hemp fiber and CBD or other plant extracts with antioxidant properties.

## Description

The subject of the invention is hemp fiber, yarn, hemp flat textile product with skin condition improving properties, clothing and a method of producing a flat textile product. More specifically, the invention concerns hemp woven fabrics, knitted fabrics and nonwovens with biological activity, which, when used in the production of clothing, improve the skin condition and slow down the visible signs of skin aging. Clothing is made of bioactive materials derived from fibrous hemp, including CBD fibers and extracts and/or hemp fiber and other plant extracts with antioxidant properties.

Hemp and linen fibers, as natural fibers, have been considered the most friendly to people for centuries. Thanks to their excellent hygienic properties, they positively affect human physiological parameters, without contributing to oxidative stress, improve the activity of the sebaceous glands, do not desynchronize muscle motor units and improve the quality of sleep. [M. Zimniewska; I. Krucińska, "The Effect of Raw Material Composition of Clothes on Selected Physiological Parameters of Human Organism" Journal of the Textile Institute, Volume 101, Issue 2, 2010, Pages 154 - 164; M. Zimniewska, I. Krucinska, Chapter 10. The Role of Apparels Based on Natural Fibers: Flax in Human Physiology and Health in Book: Natural Fibers: Properties, Mechanical Behavior, Functionalization and Applications, Editors: R.M. Kozlowski, M. Muzyczek, Nova Science Publishers, Inc., 2017, ISBN: 978-1-53612-071-4, (pp. 193-212)]. Natural hemp and flax fibers have their own biological activity, which depends on the method of their extraction from the straw and transformation processes, and enables killing of a certain number of Staphylococcus aureus and Escherichia coli bacteria. [Zimniewska M, Goślińska-Kuźniarek O. Evaluation of Antibacterial Activity of Flax Fibers Against the Staphylococcus aureus Bacteria Strain, FIBRES & TEXTILES in Eastern Europe 2016; 24, 2 (116): 120-125, DOI: 10.5604/12303666.1191439, Zimniewska, M.; Rozanska, W.; Gryszczynska, A.; Romanowska, B.; Kicinska-Jakubowska, A. Antioxidant Potential of Hemp and Flax Fibers Depending on Their Chemical Composition. Molecules 2018,23,1993; https://doi.org/10.3390/molecules23081993].

The quality of bast fibers and their bioactivity potential are closely related to the method of their extraction from fibrous plants. Fibers are obtained from plant stems as a result of the application of a multi-stage technology:
- retting - a process involving biological treatment,
- breaking - fiber isolation and extraction from the retted and dried stems,
- fiber scutching - removal of lignin-pectin incrustations from fiber by mechanical treatment.

The method of extracting the fiber from the plant stems has a great impact on the quality of the textile raw material. The following retting methods include: water retting, field retting, the so-called dew retting, chemical, enzymatic retting or retting with the use of osmose and/or ultrasounds. During the water retting process, biological decomposition of the stem takes place as a result of bacteria and enzyme action. In the first phase, glucose, polysaccharides, some pectin, tannins and protein substances are dissolved in water, and then the fermentation process in the presence of aerobic and anaerobic bacteria takes place, leading to pectin decomposition, thanks to which the stem structure is loosened and the bond between the phloem and the primary bark and lignified parts of the stem is broken. [M.A. Hann, (2005) Innovation in Linen Manufacture, Textile Progress Series, Vol. 37 No. 3, Woodhead Publishing]. Dew retting consists in leaving the harvested plants in the field and subjecting them to weather conditions: rain, sun and wind, where microorganisms, fungi and bacteria use enzymes to degrade pectin, proteins, sugars and others [Mańkowski J., 2003, The Effect of Some Agronomic Factors on the Amount and Quality of Homomorphic Fiber - Fibers & Textiles in Eastern Europe vol. 11, no. 4 (43); J.A. Rosamberg et al., 1967 Importance of Galacturonic Acid in Controlling the Retting of Flax, Appl. Microbiol., 15, 484-486].

Each of the retting methods occurs in different conditions and with the use of different active factors, therefore the obtained bast fiber is characterized by diverse properties resulting from differences in the chemical composition of the retted fibers [G. Urbańczyk, 1978, Science about the fiber, Editorial Office of the Scientific Publishers of the Lodz University of Technology].

In its chemical composition, flax and hemp fiber contains natural polymers such as cellulose, lignin, hemicellulose, pectin, waxes, fats and water-soluble substances. The average values of the individual components of flax and hemp fiber are presented in Table 1.

**Table 1. Chemical composition of flax and hemp fibers - averaged values [G. Urbańczyk, 1978, Science about the fiber, Editorial Office of the Scientific Publishers of the Lodz University of Technology]**

| Fiber | Cellulose [%] | Hemicellulose [%] | Lignin [%] | Pectin [%] | Waxes and fats [%] | Water - soluble substances [%] |
|---|---|---|---|---|---|---|
| Flax | 71.2 | 18.6 | 2.2 | 2.0 | 1.7 | 4.3 |
| Hemp | 74.4 | 17.9 | 3.7 | 0.9 | 0.8 | 2.3 |

Due to their chemical structure, hemp fibers show inherent antioxidant activity. The non-woven fabric made of these fibers, as well as the yarn from which the woven fabric/knitted fabric is then produced under a strong technological regime, retain biological activity, ensuring a positive effect on the human skin. In the present invention, long fiber hemp fabrics have been enhanced in terms of biological activity by binding of hemp panicle cannabidiol (CBD) extracts to their surfaces in the form of microcapsules or a solution directly applied to the materials. The chemical structure of cannabidiol (CBD) is shown below.

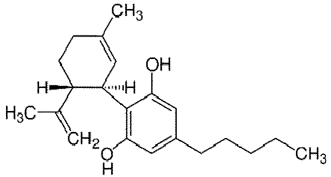

CBD is used in the production of cosmetics, as an ingredient in ointments, creams and personal care products. It has therapeutic potential, according to the literature data, cannabidiol inhibits the multiplication of keratinocytes and the formation of horny plaques in the epidermis. It has anti-inflammatory, antioxidant, antibacterial and anti-acne properties [B. Palmieri et al. A therapeutic effect of CBD-enriched ointment in inflammatory skin diseases and cutaneous scars, Clin Ter. Mar-Apr 2019; 170 (2): e93-e99. doi: 10.7417/CT.2019.2116]. Using CBD to improve the skin condition is safe and non-invasive. By the decision of the European Commission's Directorate-General for the Internal Market, Industry, Entrepreneurship and SMEs, the CBD extract was approved for the use as cosmetics component [Kacey Culliney, European Commission adds natural whole-hemp CBD to Coslng database, Cosmetic design-europe.com, 03 Feb.2021] in order to improve skin condition, to protect the skin from the harmful effects of the surrounding environment, as a substance with antioxidant activity, anti-sebum, cleansing, moisturizing and softening the skin.

Patent application CN106427109A (published on 2017-02-22) discloses a functional fabric used in the production of leisure wear, as well as the method of its preparation and use. The fabric of the invention comprises a health-care layer and a base layer. The health-care layer is made of viscose fibers prepared from cellulose obtained from fir and cinnamomum camphora as raw materials. The base layer is formed by interweaving of warp yarns and weft yarns, the warp yarns are blended yarns containing China hemp, combed cotton and fibers made of bamboo cellulose, and the weft yarns are composed of China hemp, carbon fibers and cotton. The resulting fabric is characterized by good moisture absorption, bacteriostatic properties, it provides comfort and coolness, as well as has a hygienic and health-care effect on the human body.

Patent application RO126535A2 (published on 2011-08-30) discloses a method of obtaining cellulose fabrics with antibacterial properties. The method of the invention is based on that the fabrics made of flax and hemp are subjected to a two-stage treatment, whereby in the first stage the textile carrier is grafted with a solution containing 50 to 150 g/l of monochlorotriazinyl-beta-cyclodextrin and 10 to 100 g/l of NaCO, stirred for 5 to 10 minutes, then squeezed, dried and fixed, after which the fabric is repeatedly rinsed until it reaches pH 6.5 to 7, dried and fixed at 90 to 150°. The second step comprises the formation of a complex of monochlorotriazinyl-beta-cyclodextrin with allantoin and comprises immersing the textile carrier in an aqueous solution containing 7 to 12 g/l of allantoin for 1 to 3 hours, mixing, and then removing the excess of allantoin by washing the fabric and extracting for 5 hours in water, and finally the fabric is dried at room temperature.

Patent applications CN102965792A (published on 2013-03-13) and CN203270169U (published on 2013-11-06) disclose a functional medical textile fabric with anti-bacterial functions and moisture-absorbing quick-drying functions, which is formed by interweaving warp and weft, wherein the warp is a blended yarn comprising COOLMAX fibers with chitin viscose fibers, and the weft is a blended yarn comprising cotton fiber/hemp fiber/silver fiber; and the warp and the weft adopt a plain weave structure. The selected composition of fiber blends has special durable functions of moisture absorption, quick drying, health protection, so that the textile fabric not only has good antibacterial and anti-odor properties, but also is characterized by ultra-strong moisture absorption, quick drying, static resistance, radiation resistance, size stability, washing resistance, comfortable wearing and cooling, can be used for professional medical clothing in summer, such as nurse clothes, nursing caps and patients clothes, and is an excellent textile fabric with ultra-strong antibacterial functions.

Patent application CN103504660A (published on 2014-01-15) discloses a multifunctional light absorbing fabric. The fabric is made of hemp fibers, corn fibers, ceramic nano-fibers and 100% merino wool fibers. The multifunctional light absorbing fabric comprises, by weight, 25-29% of hemp fibers, 20-24% of corn fibers, 13-18% of ceramic fibers and 29-42% of the 100% merino wool fibers. Woven multifunctional light absorbing fabric contributes to the human health care, absorbs moisture, is breathable, keeps the wearer warm and is comfortable.

Patent EP 1871948 B1 concerns microcapsules for smart textile materials, containing an active product and reactive groups, the purpose of which is chemical binding of microcapsules to the fibers. The microcapsules contain active products such as PCM (Phase Change Materials) or are characterized by controlled release of products such as fragrances, essential oils, antibacterial agents and others, to impart specific functional properties to the textile materials.

Despite the existing solutions, there is a constant need to create a hemp woven fabric/knitted fabric/non-woven, the production process of which would be designed in such a way as to ensure inherent antioxidant activity and additionally strengthen it in terms of biological activity. The existing solutions mainly concern fabrics that quickly absorb moisture, quickly dry, have antibacterial and anti-odor properties. There are also no prior art solutions that would use CBD extracts leading to hemp flat textiles showing inherent bioactive properties. There are still no solutions in which natural bioactive hemp fibers would be enriched with hemp panicle extract and would be characterized by biological activity improving skin condition.

The aim of the present invention is to create a yarn and a woven fabric/knitted fabric/non-woven fabric that retain biological activity specific to certain hemp fibers improved due to specific conditions of processes applied in the entire technological line, and clothing made of them provides a positive effect on human skin. The use of CBD extracts or other plant extracts with an antioxidant effect will enhance the bioactivity of hemp woven/knitted/non-woven fabrics, and thus enable the production of hemp clothing with biological activity, improving the skin condition and slowing down skin aging.

Surprisingly, in the present invention it was possible to create a long fiber hemp woven fabric/knitted fabric/non-woven fabric which has been improved in terms of biological activity by binding of CBD extract to the surface of fibers, e.g. by applying a solution containing CBD extract or microcapsules containing cannabidiol (CBD) extract of hemp panicle, by spraying using printing, padding or exhausting method. The solution is CBD extract in the amount of 0.5% - 3% dissolved in the oil which is the medium chain triglyceride, e.g. linseed oil, coconut oil, olive oil, grapefruit seed oil, palm oil, almond oil, avocado oil, sunflower oil. The realization of the objective thus defined and the solution of the problems described in the prior art related to the development and delivery of the invention enabling the creation of a natural fiber product, which at the same time provides a positive effect on the skin, have been achieved in the present invention.

The subject of the invention is hemp fiber with properties improving the skin condition, characterized in that the technological raw material is a long hemp fiber extracted from hemp stems by retting with the use of dew retting method and it comprises at least 60% of cellulose, at least 18% of hemicellulose, not more than 4.5% of lignin, not more than 4.0% of pectin and at least 0.4% of waxes, and its chemical composition comprises natural antioxidants, including phenolic acids, and the content of syringic acid in 100 g of fiber is 0.4-0.07 mg, p-coumaric acid - 1.5-0.2 mg/100 g fiber, ferulic acid 4.0-0.5 mg/100 g fiber, sinapinic acid 1.0-0.2 mg/100 g.

Preferably, the yarn fiber comprises at least 60% of cellulose, at least 18% of hemicellulose, no more than 4.5% of lignin, no more than 4.0% of pectin, and at least 0.4% of waxes, and it comprises natural antioxidants, including phenolic acids, where the content of syringic acid in 100 g of fiber is 0.4-0.07 mg, p-coumaric acid - 1.5-0.2 mg/100 g fiber, ferulic acid 4.0-0.5 mg/100 g of fiber, sinapinic acid 1.0-0.2 mg/100 g, and the linear weight of the yarn is not higher than 200 tex.

Another object of the invention is a hemp flat textile with biological activity improving the skin condition, characterized in that it is made of hemp fiber, which comprises at least 60% of cellulose, at least 18% of hemicellulose, not more than 3.8% of lignin, not more than 3.3% of pectin and at least 0.4% of waxes, and that it contains natural antioxidants, including phenolic acids, where the content of syringic acid in 100 g of fiber is 0.4-0.07 mg, p-coumaric acid - 1,5-0.2 mg/100 g of fiber, ferulic acid 4.0-0.5 mg/100 g of fiber, sinapinic acid 1.0-0.2 mg/100 g and the linear weight of the yarn is not higher than 200 tex.

Preferably, the surface of the flat textile is covered with plant extracts with antioxidant properties supporting the bioactivity of the hemp fibers, the plant derived substances with antioxidant effect being selected from cannabidiol, aloe, thyme and/or green tea.

Preferably, the extract of plant derived substances with an antioxidant effect is applied to the textile by direct method using padding, printing, exhausting or spraying the solution in the form of a dispersion or in the form of microcapsules with biologically active extracts, wherein the density of the microcapsule application is not less than 10 g/m².

Preferably the dispersion medium for CBD is hemp oil or other plant derived oil and the percentage of CBD in the solution is 0.5% to 3%.

Preferably, the concentration of the aloe extract is between 30% and 60%.

Preferably, the flat textile product is a product with inherent bioactivity and/or it has antibiotic effect, with the minimum MIC concentration of the hemp fabric inhibiting microbial growth being not more than 240 mg/ml, and the antibiotic activity not less than 4.2 JA/g of fabric.

Preferably, the flat textile is intended for the manufacture of clothing and it improves the skin condition and soothes changes resulting from the aging process.

Next subject of the invention is a hemp clothing with biological activity improving the skin condition, characterized in that it is made of the hemp flat textile as defined above.

Another object of the invention is a method of producing a flat textile from hemp fiber with properties improving the skin condition, characterized in that the hemp fiber is combined with plant derived extracts with antioxidant effect selected from cannabidiol, aloe, thyme and/or green tea, and the linear weight of yarn used is not higher than 200 tex.

Preferably, the extract of plant derived substances with antioxidant activity is applied to the textile by direct method using padding, printing, exhausting process or spraying of the solution in the form of a dispersion or in the form of microcapsules with biologically active extracts, and where the density of the microcapsule application is not less than 10 g/m².

Preferably the dispersion medium for CBD is hemp oil or other plant derived oil and the percentage of CBD in the solution is 0.5% to 3%.

Preferably, the concentration of the aloe extract is between 30% and 60%. Preferably, the yarn made of hemp long fiber is used to produce articles with an area weight not higher than 280 g/m².

In order to better illustrate the invention, the solution is presented in the drawing, where:
**Figure 1** is a schematic diagram of the technological chain for the production of hemp clothing with biological activity.
**Figure 2a** shows industrial/fiber hemp plants.
**Figure 2b** shows a long hemp fiber.

In order to better understand the invention, the solution is illustrated in the following examples. These examples are not intended to limit the invention, but merely allow a more precise understanding of its possible embodiments.

### Examples

### Linear products: fiber and yarn

The raw material used to produce clothing is long hemp fiber. The cultivation of hemp is focused on the production of fibers; sowing density not less than 60 kg/ha, harvest time after reaching the maturity of the fiber in the stem, after flowering, and before seed formation. The cultivation is carried out under controlled conditions that exclude the use of pesticides and other chemical plant protection products that could penetrate the fiber structure, reducing their therapeutic potential. After reaching maturity of the fibers in the stem plants are cut and then subjected to the dew retting process. As the effect of weather conditions, rainfall and sunlight, the phloem is detached from the woody part of the plant, pectin are decomposed, allowing the fiber to be extracted from the stems.

The extracted hemp fiber contains approximately 66% of cellulose, 22% of hemicellulose, 4.3% of lignin, 3.7% of pectin and 0.56% of waxes. The chemical composition includes phenolic acids, which are natural antioxidants. The content of syringic acid in 100 g of fiber is 0.4-0.07 mg, p-coumaric acid - 1.5-0.2 mg/100 g of fiber, ferulic acid 4.0-0.5 mg/100 g of fiber, sinapinic acid 1.0-0.2 mg/100 g.

The flax processing technology was adapted for the transformation of hemp into fiber and yarns for clothing products, as there is no hemp spinning system. The process parameters and the working elements of the machines in the technological line were adapted to the needs of hemp processing. After the process of breaking and scutching, the fiber with a length of approximately 75-100 cm in the form of a handful is subjected to the combing process appropriate for long fiber using a combing machine suitable for hemp processing. After the strip is formed, the fiber flux, evened and thinned on splicers and stretchers, is sent to the roving machine to form a roving. Yarns with a linear weight not higher than 85 tex are formed from roving by wet spinning with the use of the linen machine park adapted to hemp processing.

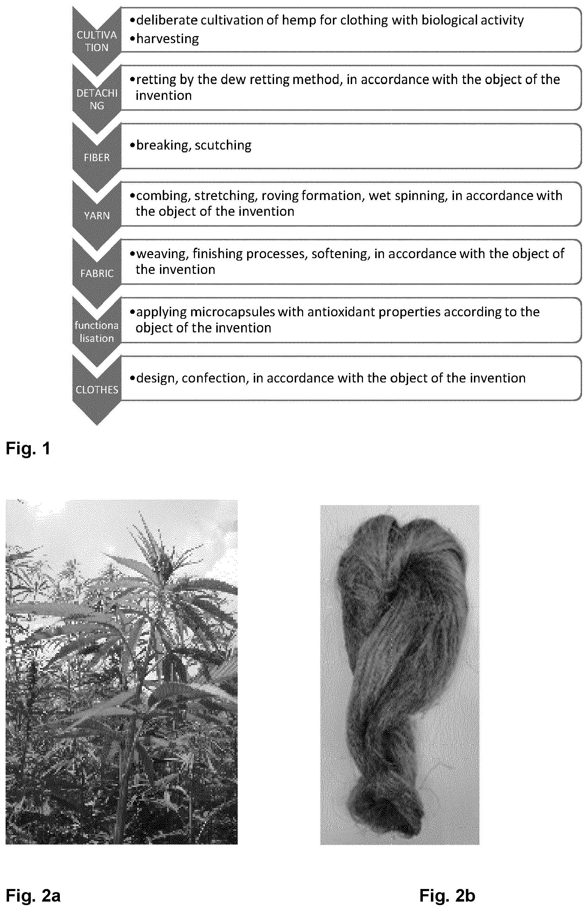

A schematic illustration of the value chain for the production of hemp clothing with biological activity is shown in Fig. 1.

### Textiles

Fabrics with the surface weight not higher than 280 g/m² are made of hemp long fiber yarn. The fabrics undergo a mechanical softening process through tamboring. No chemicals are used during the fabric finishing process.

Fabrics made of hemp fiber contain in their chemical composition antioxidants in the form of phenolic acids: syringic acid in the range of 0.7-0.15 mg in 100 g of fabric, p-coumaric acid 0.8-0.1 mg in 100 g of fabric, ferulic acid1,5-0.5 mg in 100 g of fabric. Fabrics do not show cytotoxic activity, but show a slight antibiotic effect: MIC - the lowest concentration of the biocide (in this case, hemp fabric) inhibiting the growth of microorganisms, is not more than 240 mg/ml, the antibiotic activity is not less than 4.2 JA/g of fabric.

Microcapsules containing cannabidiol CBD extracts or extracts obtained from other herbal plants with antioxidant properties are applied to the surface of the fabrics produced. CBD extracts do not have a psychotropic effect as they do not contain illicit THC substances and are allowed for use in cosmetic preparations. Microcapsules with biologically active extracts are applied to the surface of the fabric using the padding method, the microcapsule application density is not less than 10 g/m². The CBD extract can also be applied to a woven/knitted/non-woven fabric by direct method using padding, printing, spraying the solution in the form of a dispersion or using exhausting process, with hemp oil or other plant-derived oil being the dispersion medium for CBD. The percentage of CBD in the solution is about 0.5%-3%.

The table below shows the concentration values of the CBD extract in the oil applied on woven/knitted/non-woven fabric to achieve a positive effect on human skin.

**Table 2**

| Application method | Padding | Printing | Spraying method | Exhausting process | Microcapsule Application density |
|---|---|---|---|---|---|
| CBD extract concentration in oil | 1.5%-3.0% | 0.5%-2.0% | 0.5%-2.0% | 1.5%-3.0% | > 10 g/m² |
| Aloe extract concentration | 30%-60% | 20%-40% | 30%-60% | 30%-60% | > 10 g/m² |

### Example 1:

In order to make clothing with antioxidant properties, hemp fiber was obtained - from the hemp culture with a sowing density of 60 kg/ha. In order to extract the fiber from the hemp stems, field retting - dew retting - was used. The content of natural phenolic acid antioxidants in the fiber is presented in Table 3.

**Table 3. The content of phenolic acids in the hemp fiber.**

| | | | | | | |
|---|---|---|---|---|---|---|
| Long hemp fiber | Syringic acid content [mg/100 g] | | Sinapinic acid content [mg/100 g] | | p-coumaric acid content [mg/100 g] | |
| | Result | SD | Result | SD | Result | SD |
| | 0.035 | 0.001 | 0.608 | 0.184 | 0.034 | 0.002 |

Long hemp fiber contains phenolic acids, which are natural antioxidants.

A yarn with the linear weight of 83 tex was made from long hemp fiber using the technological line intended for spinning long flax fibers, after its previous adaptation to the parameters of the hemp fiber.

A fabric with the area weight of 267 g/m² was produced from the yarn. The process of removing size and other contaminants from fabrics was carried out under mild conditions of standard washing. Evaluation of the biological activity potential of the hemp fabric is presented in Tables 4 and 5.

**Table 4. Evaluation of biological potential of hemp fabric made of yarn with linear weight of 83 tex; natural color in raw state; plain weave; area weight of 267 g/m².**

| | | | | | | | | | |
|---|---|---|---|---|---|---|---|---|---|
| Raw fabric | Syringic acid content [mg/100 g] | | p-coumaric acid content [mg/100 g] | | Ferulic acid content [mg/100 g] | | Cytotoxicity [%] | MIC [mg/ml] | JA/g |
| | Result | SD | Result | SD | Result | SD | | | |
| | 0.178 | 0.008 | 0.013 | 0,000 | 0.645 | 0.007 | No toxic effect | 240 | 4.2 |

**Table 5. Antioxidant activity of hemp fabric determined by FRAP method**

| Trial name | Trial concentration mg/ml | FRAP µmol/L | Average FRAP µmol/L | SD |
|---|---|---|---|---|
| Hemp fabric | 3.025 | 151.45 | 149.80 | 14.86 |
| | | 134.18 | | |
| | | 163.76 | | |

The raw fabric made of long hemp fibers contains inherent phenolic acids in its chemical composition, does not show any toxic effect in contact with the skin, has a slight antibiotic effect and a significant antioxidant effect.

The CBD extract was applied using the padding method. Cannabidiol has a proven antioxidant and anti-inflammatory potential, thanks to which CBD or microcapsules with CBD placed on the inner surface of clothing support the bioactive effect of hemp fiber through direct contact with the skin.

### The effect of wearing hemp clothes on human skin

The effect of wearing hemp clothes on human skin was investigated on the basis of trials of wearing these clothes, which lasted for 6 weeks. The studies included the assessment of the dermatological condition and the measurement of biophysical parameters of the skin in 15 female volunteers aged 21 to 79 years. The probands were required to wear hemp clothes with applied microcapsules during the whole day of activity. None of the volunteers reported the occurrence of skin diseases, and the skin condition was described as normal.

Three types of studies were carried out in volunteers:
- TEWL test - tevametry (trans *epidermal water loss*);
- corneometry;
- sebometry - seborrhoea testing.

Tevametry is a non-invasive method of testing the level of transepidermal water loss, which is an extremely important parameter in the assessment of skin hydration. The higher the TEWL value, the more the epidermal barrier is weakened. In the case of older people, the skin is often very dry - stratum corneum hyperplasia (hyperkeratosis) often causes reduced transepidermal water loss, moreover, reduced activity of sweat glands and a slower process of self-exfoliation of the skin is observed with age, reducing the level of NMF (natural moisturizing factor). [https://www.symbiosis.pl/konsultacje/badanie-tewl]

Corneometry is a method of testing the hydration of the epidermis - the outermost layer of the skin. This method is based on the phenomenon of dependence of the epidermis electrical resistance on its hydration - the higher the water content, the lower the electrical resistance of tissues. Based on the electrical resistance of the tissue, it is possible to calculate the degree of its hydration. [http://www.dermatopedia.pl/art/2013pl003.html]

Testing of seborrhea - sebumetry makes it possible to assess the condition of skin lubrication and the activity of sebaceous glands, mainly on the face and scalp. The sebumeter works by photometric measurement of the transparency of a special tape that was pressed against the skin during the test. The device analyzes the tape soaked with fat that has been accumulated on the surface of the epidermis - it assesses the light transmission through the paper and displays the result. [https://www.symbiosis.pl/konsultacje/badanie-lojotoku] Sebumetry allows you to assess the condition of skin lubrication and the activity of sebaceous glands.

**Table 6. Tevametry results before and after 6 weeks of wearing textiles with CBD microcapsules.**

| Microcapsule type | TEWL (g/m²/hour) M ±SD | | |
|---|---|---|---|
| | before | after | difference |
| CBD | 8.1 ± 1.2 | 9.8 ± 2.9 | 1.8 ± 2.8 |

Clothing with CBD microcapsules slightly increased the epidermis water loss.

**Table 7. Comparison of corneometry results before and after 6 weeks of wearing textiles with microcapsules containing CBD.**

| Microcapsule type | Corneometry (AU) M ±SD | | |
|---|---|---|---|
| | before | after | difference |
| CBD | 46.5 ± 8.6 | 56.2 ± 8.0 | 9.7 ± 9.9 |

As shown in Table 6, clothing enriched with microcapsules containing CBD caused a slight increase in skin hydration, and therefore had a positive effect on maintaining the proper function of the epidermal barrier.

**Table 8. Sebumetry results before and after 6 weeks of wearing textiles with CBD microcapsules.**

| Microcapsule type | Sebumetry M ±SD | | |
|---|---|---|---|
| | before | after | difference |
| CBD | 2.0 ± 2.8 | 3.2 ± 3.0 | 1.2 ± 4.5 |

A sebumetric study showed that microcapsules with CBD extract slightly increased sebum secretion.

### Conclusion:

Research confirms the beneficial effect of clothing with CBD microcapsules on users skin, which may improve the skin condition and slow down the appearance of signs of skin aging with long-term systematic use.

## Claims

1. A hemp fiber with properties improving skin condition, **characterized in that** the technological raw material is a long hemp fiber extracted from hemp stems by retting with the use of dew retting method, and it comprises at least 60% of cellulose, at least 18% of hemicellulose, not more than 4.5% of lignin, no more than 4.0% of pectin and at least 0.4% of waxes, and its chemical composition comprises natural antioxidants, including phenolic acids, wherein the content of syringic acid in 100 g of fiber is 0.4-0,07 mg, p-coumaric acid - 1.5-0.2 mg/100 g of fiber, ferulic acid 4.0-0.5 mg/100 g of fiber, sinapinic acid 1.0-0.2 mg/100 g.

2. A hemp fiber yarn, **characterized in that** the yarn fiber comprises at least 60% of cellulose, at least 18% of hemicellulose, not more than 4.5% of lignin, not more than 4.0% of pectin and at least 0.4% of waxes, and that it comprises natural antioxidants, including phenolic acids, wherein the content of syringic acid in 100 g of fiber is 0.4-0.07 mg, p-coumaric acid 1.5-0.2 mg/100 g fiber, ferulic acid 4.0-0.5 mg/100 g of fiber, sinapinic acid 1.0-0.2 mg/100 g, and the linear weight of the yarn is not higher than 200 tex.

3. A hemp flat textile product with biological activity improving the skin condition, **characterized in that** it is made of hemp fiber, which comprises at least 60% of cellulose, at least 18% of hemicellulose, no more than 3.8% of lignin, no more than 3.3% of pectin and at least 0.4% of waxes, and it comprises natural antioxidants, including phenolic acids, wherein the content of syringic acid in 100 g of fiber is 0.4-0.07 mg, p-coumaric acid - 1.5-0.2 mg/100g of fiber, ferulic acid 4.0-0.5 mg/100 g of fiber, sinapinic acid 1.0-0.2 mg/100 g and the linear weight of the yarn is not higher than 200 tex.

4. The flat textile product according to claim 3, **characterized in that** its surface is covered with plant extracts with antioxidant properties supporting the bioactivity of hemp fibers, wherein plant derived substances with antioxidant activity are selected from cannabidiol, aloe, thyme and/or green tea.

5. The flat textile product according to any one of claims 3 to 4, **characterized in that** the extract of plant derived substances with an antioxidant effect is applied to the textile by direct method using padding, printing, exhausting or spraying of the solution in the form of a dispersion or in the form of microcapsules with biologically active extracts, wherein the application density of the microcapsules is not less than 10 g/m².

6. The flat textile product according to any one of claims 3 to 5, **characterized in that** the dispersion medium for CBD is hemp oil or other plant derived oil and the percentage of CBD in the solution is 0.5% to 3%.

7. The flat textile product according to any one of claims 3 to 6, **characterized in that** the concentration of the aloe extract is between 30% and 60%.

8. The flat textile product according to any one of claims 3 to 7, **characterized in that** it is a product of inherent bioactivity and/or it exhibits antibiotic activity, wherein the minimum MIC concentration of the hemp fabric inhibiting microbial growth is not more than 240 mg/ml, and the antibiotic activity is not less than 4.2 JA/g of fabric.

9. The flat textile product according to any one of claims 3 to 8, **characterized in that** it is intended for the manufacture of clothing and it improves the skin condition and soothes changes resulting from the aging process.

10. A hemp clothing with skin-improving biological activity **characterized in that** it is made of the hemp flat textile product as defined in claims 3 to 9.

11. A method for producing the flat textile product from hemp fiber with skin-improving properties, **characterized in that** the hemp fiber is combined with plant derived extracts with antioxidant activity selected from cannabidiol, aloe, thyme and/or green tea, and a yarn with a linear weight not higher than 200 tex is used.

12. The method for producing the flat textile product according to claim 11, **characterized in that** the extract of plant derived substances with an antioxidant effect is applied to the textile product by direct method using padding, printing, exhausting or spraying a solution in the form of a dispersion or in the form of microcapsules with biologically active extracts, and where the application density of the microcapsules is not less than 10 g/m².

13. The method for producing the textile flat product according to any one of claims 11 to 12, **characterized in that** the dispersion medium for CBD is hemp oil or other plant derived oil and the percentage of CBD in the solution is 0.5% to 3%.

14. The method for producing the textile flat product according to any one of claims 11 to 13, **characterized in that** the concentration of the aloe vera extract is between 30% and 60%.

15. The method for producing the textile flat product according to any one of claims 11 to 14, **characterized in that** the yarn made of hemp long fiber is used to produce products with an area weight not higher than 280 g/m².
